# EUROPÄISCHE PATENTANMELDUNG

(11) **EP 0 565 906 A2**
(43) Veröffentlichungstag der Anmeldung: **20.10.1993**
(21) Anmeldenummer: 93104610.6
(22) Anmeldetag: 20.03.1993
(51) Int. Cl.: G01N 33/36

(54) **Verfahren und Vorrichtung zum Erfassen von Längenungleichmässigkeiten der einzelnen Garnkomponenten eines Zwirns**

(30) Priorität: 14.04.1992 DE 4212467
(71) Anmelder: Saurer-Allma GmbH, D-87437 Kempten (DE)
(72) Erfinder: de Vuyst, B-87901 Waregem (BE); Vanstaen,Roland, B-Kortrijk (BE); Lorenz,Rainer, W-4054 Nettetal-Breyell (DE)
(74) Vertreter: Sroka, Peter-Christian, Dipl.-Ing.

(57) **Zusammenfassung**

Die Qualität eines Zwirns wird im wesentlichen danach beurteilt wie gleichmäßige die Fadenlängen der einzelnen Garnkomponenten des Zwirns in diesen eingebettet sind. Zur Erfassung von Längenungleichmäßigkeiten der einzelnen Garnkomponenten eines Zwirns werden ein Verfahren und eine Vorrichtung vorgeschlagen, bei dem bzw. bei der der Zwirn in seiner Längsrichtung mit konstanter Geschwindigkeit zwischen einem Lichtsender und einem Lichtempfänger einer opto-elektronischen Meß- und Auswerteinrichtung hindurchgeführt wird, die auf Änderungen der Zwirnstruktur in Längsrichtung reagiert und ein Ausgangssignal abgibt, dessen zeitlicher Verlauf eine Funktion der abgetasteten Strukturänderungen darstellt, so daß dieses Ausgangssignal analysiert und auf periodische Komponenten untersucht und daraus die Zwirnperiode und/oder weitere, die Struktur des Zwirns charakterisierende Parameter bestimmt werden können, wobei man in das von dem Lichtsender ausgesandte Strahlenbündel vor oder hinter dem Zwirn eine Schlitzblende einführt und den Schlitz dieser Blende relativ zur Bewegungsrichtung des Zwirns schrägstellt, vorzugsweise parallel zum Drehungs- bzw. Zwirnsteigungswinkel α des zu untersuchenden Zwirns.

## Beschreibung

Die Erfindung betrifft ein Verfahren und eine Vorrichtung zum Erfassen von Längenungleichmäßigkeiten der einzelnen Garnkomponenten eines Zwirns, bei dem bzw. bei der der Zwirn in seiner Längsrichtung mit konstanter Geschwindigkeit zwischen einem Lichtsender und einem Lichtempfänger einer opto-elektronische Meß- und Auswerteinrichtung hindurchgeführt wird, die auf Änderungen der Zwirnstruktur in Längsrichtung reagiert und ein Ausgangssignal abgibt, dessen zeitlicher Verlauf eine Funktion der abgetasteten Strukturänderungen darstellt, so daß dieses Ausgangssignal analysiert und auf periodische Komponenten untersucht und daraus die Zwirnperiode und/oder weitere, die Struktur des Zwirns charakterisierende Parameter, bestimmt werden können.

Für die Qualität eines Zwirns gelten bestimmte Standardnormen. Für bestimmte Zwirne ist es dabei eine wesentliche Voraussetzung, daß die einzelnen zu dem Zwirn vereinigten Garnkomponenten über die Länge des fertigen Zwirns die gleichen Längen haben, d.h. es ist gefordert, daß eine Längengleichheit zwischen den Einfachgarnkomponenten eines Zwirnes vorliegt.

Bei einem bekannten, in der DE 36 28 654 A1 beschriebenen Verfahren wird der Zwirn in seiner Längsrichtung mit konstanter Geschwindigkeit zwischen einem Lichtsender und einem Lichtempfänger einer opto-elektronischen Auswerteinrichtung hindurchgeführt, wobei das von dem Lichtsender ausgesendete Strahlenbündel mittels geeigneter Blenden so abgeschirmt wird, daß das Strahlenbündel nur streifend auf den Zwirn auftrifft und damit nur einseitig zur Längsachse des Zwirns. Der am Zwirn vorbeigelassene Anteil des Strahlenbündels trifft auf den Lichtempfänger, beispielsweise eine Fotodiode oder einen Fototransistor, und die Ausgangssignale des Lichtempfängers werden einer Auswerteinrichtung zugeführt, in der diese Signale auf periodische Komponenten untersucht und analysiert werden. Wenn der Zwirn beispielsweise eine gegenüber der oder den anderen Garnkomponenten längere Einfachgarnkomponente aufweist, hat dieses periodisch synchron mit der Zwirnperiode auftretende, seitliche Ausbuchtungen zur Folge, die zu entsprechenden periodischen Veränderungen der auf den Lichtempfänger auftreffenden Lichtmenge führen.

Bei dem bekannten Verfahren besteht eine Schwierigkeit darin, den Zwirnfaden mittels der Blenden in geeigneter Weise abzuschatten, da beispielsweise für die gemäß diesem bekannten Verfahren vorgeschlagene Halbabschattung des Zwirns sehr straffe Führungsmittel für den Zwirn notwendig sind. Da der Zwirn nur einen begrenzten Durchmesser hat, kann es vorkommen, daß selbst geringste Fadenschwingungen in der Meßstrecke zur Abschattung übriger Querschnittsbereiche am Zwirn führen und so ein verfälschtes Ergebnis erhalten wird.

Ein in der CH-6 75 133 A5 beschriebenes System zur Messung der Drehung z.B. eines Garnes beruht auf der Messung der von dem Garn reflektierten Lichtstrahlen, die von zwei Lichtquellen schräg zur Garnlaufrichtung ausgesandt werden. Um zuverlässige Meßergebnisse zu erhandeln, ist eine exakte Führung des Garns erforderlich, d.h. es bestehen die gleichen Schwierigkeiten, wie sie oben beschrieben sind.

Die DE-1 560 540 A1 behandelt ein System zur Überwachung eines laufenden Fadens auf Dick- oder Dünnstellen mit einer zwischen einem Lichtsender und einem Lichtempfänger angeordneten Schlitzblende, deren Schlitz mindestens annähernd senkrecht zur Fadenlaufrichtung liegen soll. Eine korrekte Dick- oder Dünnstellenanalyse kann aber immer nur dann richtig erfolgen, wenn der Schlitz senkrecht zur Fadenlaufrichtung liegt. Es ist zwar denkbar, eine Dick- oder Dünnstellenerfassung durch einen schrägen Schlitz zu erzielen. In einer solchen Anordnung kann aber kein Sinn gesehen werden, da bei einer schrägen Schlitzstellung in einem Teilbereich der Fadenlängsausdehnung ein "gesunder" Fadenbereich erfaßt wird und in dem schräg gegenüberliegenden Abschnitt bereits ein Dünn- oder Dickstellenbereich. Es müßte eine erhebliche Sensibilität in der Auswerteelektronik angewandt werden, um die nicht gewünschte Größenordnung einer Dick- oder Dünnstelle herauszufiltern. Außerdem sind Dick- und Dünnstellen in einem Garnstrang nie von so gleichmäßiger Struktur, daß nicht auch eine Ausbauchung an einer Fadenseite mit einer Einbuchtung der schräg gegenüberliegenden Fadenseite zusammenfällt, wobei in der Summe der abgeschatteten Flächenintegration der Elektronik wieder ein voller Fadenquerschnitt "vorgegaukelt" wird. Auch dieses System ist entsprechend mit Fehlern behaftet.

In der EP 0 423 380 A1 ist eine Vorrichtung zum Messen von Unregelmäßigkeiten von Fäden an einer Textilmaschine mittels einer opto-elektronischen Einrichtung beschrieben. Bei dieser Einrichtung wird der zu prüfende Faden zwischen einem Lichtsender und einem Lichtempfänger durch einen Meßschlitz hindurchgeführt, wobei im Meßschlitz zusätzlich ein Kondensatorbelag angebracht ist, der mit einer kapazitiven Meßeinrichtung verbunden ist. Mit Hilfe einer solchen Meßeinrichtung soll sowohl der Fadendurchmesser als auch die Fadenmasse gleichzeitig gemessen werden, wobei diese Meßwerte ein und derselben Stelle auf einem Faden zuzuordnen sind.

Die EP 0 118 466 B1 beschreibt ein kontinuierliches, zerstörungsfreies Verfahren zur Bestimmung der Drehung eines durch eine Meßvorrichtung hindurchbewegten Zwirns. Bei diesem Verfahren wird die Vibrationsfrequenz einer Schneide gemessen, die mit einer Kante mit dem laufendem Zwirn in Kontakt ist, wobei die Drehungszahl des Zwirns als eine Funktion der Vibrationsfrequenz dieser Schneide errechnet wird. Diese Messerschneide ist verschwenkbar bzw. drehbar, um die mit dem Zwirn in Kontakt stehende Kante parallel zum Zwirn - bzw. Verdrehungswinkel α des Zwirnes auszurichten. Dieses bekannte zur Bestimmung der Drehung bzw. der Drehungsdichte des Zwirnes bestimmte Verfahren eignet sich nicht für die Messung von Unregelmäßigkeiten im Zwirn, die beispielsweise durch unterschiedliche Längen der einzelnen Einfachgarnkomponenten bedingt sind.

Ausgehend von dem in der DE 36 28 654 A1 beschriebenen opto-elektronischen Abtastverfahren besteht die der Erfindung zugrunde liegende Aufgabe darin, bei einem derartigen opto-elektronischen Meßverfahren Maßnahmen vorzusehen, die bei einem den ganzen Zwirnquerschnitt umflutenden Lichtbündel sowie einem nicht präzise zur Schlitzblende ausgerichteten Fadenlauf und bei einem in der Meßstrecke schwingenden Faden zu reproduzierbaren und aussagekräftigen Ergebnissen darüber führen, ob der zu untersuchende Zwirn insbesondere durch Längenungleichmäßigkeiten der einzelnen Garnkomponenten bedingte Unregelmäßigkeiten aufweist.

Zur Lösung dieser Aufgabe ist das erfindungsgemäße Verfahren dadurch gekennzeichnet, daß man in das von dem Lichtsender ausgesandte Strahlenbündel zwischen dem Lichtsender und dem Lichtempfänger vor oder hinter dem Zwirn eine das Strahlenbündel schneidende Schlitzblende einfügt und den Schlitz dieser Blende relativ zur Bewegungsrichtung des Zwirns schrägstellt. Wenn eine Einfachgarnkomponente im fertigen Zwirn länger ist als eine andere Einfachgarnkomponente, wird diese längere Einfachgarnkomponente in Richtung des Steigungswinkels α des Zwirns, der dem Steigungswinkel einer Schraube entspricht, eine größere Erstreckung haben als eine kürzere Einfachgarnkomponente. Durch entsprechendes Schrägstellen des Schlitzes der vor oder hinter dem Zwirn angebrachten Blende lassen sich infolge Längenungleichmäßigkeiten der einzelnen Einfachgarnkomponenten bedingte Abweichungen selbst bei Fadenschwingungen innerhalb der Meßstrecke genau feststellen, da unabhängig von der jeweiligen Lage eines relativ zu der Schlitzblende momentan orientierten Fadenlaufs entsprechend den Längenungleichmäßigkeiten periodisch variierende Lichtmengen auf den Lichtempfänger auftreffen. Eine Voraussetzung dafür ist es, daß der Schlitz eine ausreichende Länge hat, so daß gewährleistet ist, daß ein in der Meßstrecke gegebenfalls schwingender Faden nicht über eines der beiden Schlitzenden ausschwingen kann.

Während bei einem "gleichmäßigen" Zwirn mit gleichlangen Einzelgarnkomponenten keine außergewöhnliche Modulation der festgestellten Signale auftreten, lassen sich Ungleichmäßigkeiten bei schräggestelltem Schlitz sofort feststellen, selbst dann, wenn Veränderungen der Relativlage zwischen Schlitz und Zwirn während der Messung auftreten sollten.

Bei Längenungleichmäßigkeiten erreichen die periodischen Schwankungen ein Maximum, wenn man den Schlitz übereinstimmend mit bzw. parallel zu dem Zwirn-Steigunswinkel α des zu untersuchenden Zwirns schrägstellt.

Die erfindungsgemäße Vorrichtung ist gekennzeichnet durch eine zwischen dem Zwirn und dem Lichtempfänger oder zwischen dem Zwirn und dem Lichtsender angeordnete Schlitzblende, deren vorzugsweise rechteckiger Schlitz relativ zur Bewegungsrichtung des Zwirns schräggestellt ist.

Die Blende ist vorzugsweise um eine auf der Längsachse des die Meßstrecke durchlaufenden Zwirns senkrecht stehende Achse verstellbar bzw. drehbar, um den Blendenschlitz für Zwirne unterschiedlicher Drehung bzw. unterschiedlichen Zwirnsteigerungswinkel α jeweils in die optimale Lage bringen zu können, in der der Blendenschlitz parallel zum Zwirnsteigerungswinkel α liegt

Die opto-elektronische Auswerteinrichtung ist vorzugsweise an geeigneter Stelle im Fadenlauf einer zwirnerzeugenden Maschine integriert, die die einzelnen Einheiten wie Lichtsender, Lichtempfänger und elektronische Komponenten enthält. Die Auswerteeinheit kann dann über Kabel verbunden an anderer geeigneter Stelle der Maschine untergebracht werden. Sehr vorteilhaft kann die Meßeinrichtung auch als handgehaltenes Gerät ausgeführt werden, da gerade in diesem Fall eine präzise Orientierung von Fadenlauf zu Meßschlitz nicht zu ermöglichen ist.

Um mit einer derartigen Vorrichtung ohne größere Verstellungen der Schlitzblende Messungen sowohl bei Zwirnen mit Z-Drehung als auch mit S-Drehung durchführen zu können, hat die Blende zwei sich vorzugsweise rechtwinklig kreuzende Schlitze.

Bezogen auf einen Durchmesser d des Zwirns soll die Schlitzhöhe vorzugsweise im Bereich des 1-3 fachen der Zwirndicke liegen. Bezogen auf die Drehung und die gewünschte Auflösung sollte die Schlitzbreite vorzugsweise das 0,05- bis 0,15-fache der Zwirndrehungsperiode, also üblicherweise 0,1-0,3 mm betragen.

Im folgenden wird anhand der beigefügten Zeichnungen ein Ausführungsbeispiel für das erfindungsgemäße Verfahren und eine Vorrichtung zur Durchführung des Verfahrens näher erläutert.
In den Zeichnungen zeigt:
- Figur 1: in stark vergrößerter Darstellung einen Abschnitt eines Zwirns mit S-Drehung bei gleicher Länge der Zwirnkomponenten;
- Figur 2: in einer Darstellung analog zu Figur 1 einen Zwirn bei ungleicher Länge der beiden Zwirnkomponenten;
- Figur 3: in einer Darstellung analog zu Figur 2 einen Zwirn mit Z-Drehung bei ungleicher Länge der beiden Zwirnkomponenten;
- Figur 4: eine schematische Darstellung der erfindungsgemäßen opto-elektronischen Meßeinrichtung;
- Figur 5: in perspektivischer Darstellung eine Schnittansicht einer als Handgerät ausgeführten opto-elektronischen Meß- und Auswerteinrichtung;
- Figur 6: in vergrößerter perspektivischer Darstellung eine Schnittansicht der erfindungsgemäß verwendeten Schlitzblende.

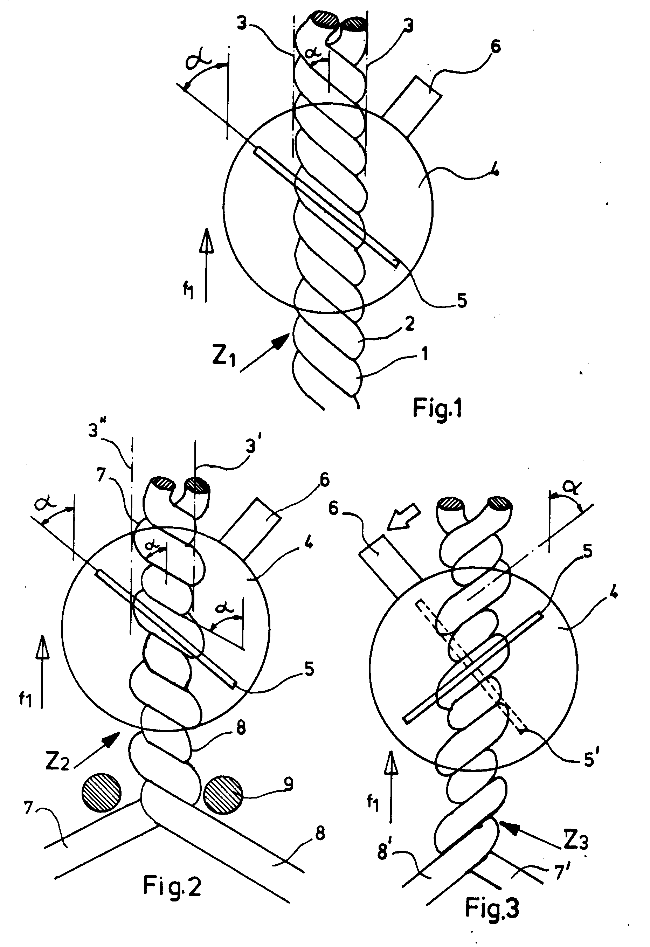

Figur 1 zeigt in einer schematisierten Darstellung die Struktur eines Zwirns Z1 aus den beiden Zwirn- bzw. Einfachgarnkomponenten 1 und 2, die beide die gleiche Länge aufweisen. Der Winkel α , der z.B. bei Cordzwirn üblicherweise zwischen 10° und 30° variiert, entspricht dem Drehungs- bzw. Zwirnsteigungswinkel entsprechend dem Steigungswinkel bei einem Schraubengewinde.

Eine in Figur 1 schematisiert dargestellte Blende 4 ist mit einem vorzugsweise rechteckigen Längsschlitz 5 versehen, der mittels eines an der Blende 4 angebrachten Einstellelementes 6 in Übereinstimmung mit dem Drehungs- bzw. Zwirnsteigungswinkel α parallel zu diesem ausgerichtet werden kann.

Wenn man ein derartiges aus dem Zwirn Z1 und der Blende 4 bestehendes System zwischen einem Lichtsender und einem Lichtempfänger anordnet, ist es offensichtlich, daß von einem von dem Lichtsender ausgesandten Strahlenbündel bei jeder Schlitzlage jeweils die gleiche Lichtmenge auf den Lichtempfänger auftrifft, unabhängig davon, wie oder ob der Blendenschlitz 5 von der Einfachgarnkomponente 1 oder der Einfachgarnkomponente 2 abgedeckt wird. Dieses gilt auch für den Fall, daß der Zwirn Z1 innerhalb der Meßstrecke relativ zur Blende 4 quer zu seiner Längsachse ausschwingt, solange gewährleistet ist, daß der ausschwingende Zwirn Z1 nicht über eines der beiden Enden des Blendenschlitzes 5 ausschwingt.

Die bei einem gleichmäßigen Zwirn Z1 auf den Lichtempfänger auftreffenden Lichtmengen sind immer gleichmäßig und unterliegen nur den periodischen Schwankungen, abgesehen von den herausfilterbaren stochastichen Störungen, die durch die Einbuchtungen zwischen benachbarten gleichlangen Garnkomponenten 1 und 2 bedingt sind.

Bei dem in Figur 2 dargestellten Zwirn Z2 hat die Garnkomponente 7 eine größere Länge als die Garnkomponente 8, was im Bereich der Garnkomponente 7 zu erkennbaren Ausbuchtungen an der Zwirnoberfläche führt. Bezüglich des Drehungs- oder Zwirnwinkels α und dem Blendensystem 4, 5, 6 wird auf die Ausführungen zu Figur 1 hingewiesen.

Es ist offensichtlich, daß, wenn der Zwirn Z2 und die parallel zum Zwirnsteigungswinkel α orientierte Blende 4 sich in einer Meßstrecke zwischen einem Lichtsender und einem Lichtempfänger befinden von einem von dem Lichtsender ausgesandten Strahlenbündel unterschiedliche Lichtmengen durch den Blendenschlitz 5 hindurchtreten bzw. abgeschattet werden, und zwar abhängig davon, ob sich der Blendenschlitz 5 hauptsächlich im Bereich der Garnkomponente 7 oder der Garnkomponente 8 befindet. Die periodisch mit der durchlaufenden Zwirnperiode schwankenden, auf den Lichtempfänger auftreffenden Lichtmengen dienen als Meßgröße für Längenungleichmäßigkeiten zwischen den beiden Garnkomponenten 7 bzw. 8.

Figur 2 zeigt außerdem ein Fadenführungselement 9.

Der in Figur 3 schematisiert dargestellte Zwirn Z3 hat eine Z-Drehung und besteht aus zwei ungleichmäßig langen Garnkomponenten 7' bzw. 8', woraus in Übereinstimmung mit Figur 2 im Bereich der längeren Garnkomponente 7' erkennbare Ausbuchtungen an der Zwirnoberfläche resultieren. Zur Messung bzw. Erfassung von Längenungleichmäßigkeiten bei einem derartigen Zwirn Z3 mit Z-Drehung wird die Blende 4 vorteilhaft mittels des Einstellelementes 6 so verstellt, daß der Blendenschlitz 5 bevorzugt parallel zum Drehungs- bzw. Zwirnsteigungswinkel α liegt. Zur Vereinfachung der Messung kann die Blende 4 statt eines Einstellmechanismus auch eine feste Orientierung zum Fadenlauf und zusätzlich einen weiteren Längsschlitz 5' aufweisen, der den Längsschlitz 5 in der ideellen Längsachse des in Richtung des Pfeiles F1 bewegten Zwirn Z3 schneidet. Mit einem solchen Kreuzschlitz sind mit einer festen Einrichtung Zwirne mit unterschiedlicher Drehung und unterschiedlichem Drehsinn zu vermessen.

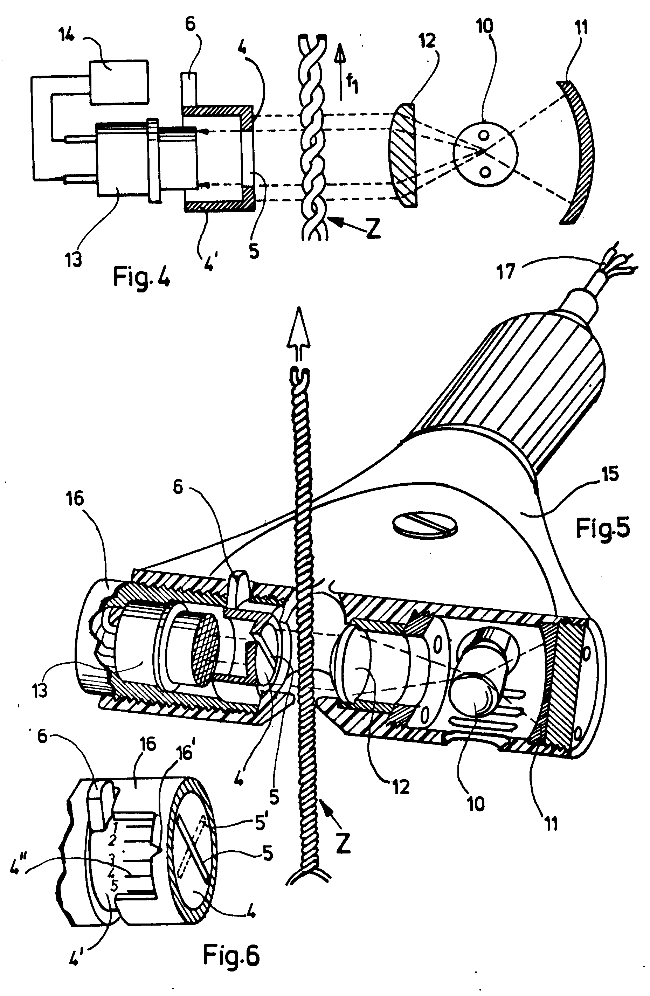

Die in Figur 4 schematisiert dargestellte erfindungsgemäße Vorrichtung besteht aus an einem an sich bekannten Lichtsender, beispielsweise in Form einer Lichtquelle 10, eines Spiegels 11 und einer Linse 12. Wesentlich ist, daß diese Lichtquelle 10, 11, 12 in der Lage ist, ein gebündeltes Strahlenbündel auszusenden. Als Lichtempfänger kann in bekannter Weise eine Fotodiode oder ein Fototransistor 13 gegebenenfalls mit einer weiteren Optik verwendet werden. Die Empfängersignale werden dann in bekannter Weise in einer an sich bekannten Auswerteeinheit 14 verarbeitet. Bei dem Lichtsender 10 - 12, dem Lichtempfänger 13 und dem Auswerter 14 handelt es sich um eine übliche opto-elektronische Einrichtung, die für sich allein betrachtet nicht Gegenstand der vorliegenden Erfindung ist.

In der Meßstrecke zwischen dem Lichtsender 10, 11, 12 und dem Lichtempfänger 13 befindet sich die mittels des Einstellelementes 6 verstellbare Blende 4, die in der in Verbindung mit den Figuren 1 - 3 beschriebenen Weise einen Längsschlitz 5 aufweist. Wenn ein zu untersuchender Zwirn Z in Richtung des Pfeiles f1 durch die Meßstrecke hindurchbewegt wird, führt dieses zu einer entsprechenden Abschattung des durch den Blendenschlitz 5 auf den Lichtempfänger 13 auftreffenden Strahlenbündels. Wenn es sich bei den Zwirn Z um einen gleichmäßigen Zwirn etwa gemäß Figur 1 handelt, findet nur eine schwellperiodische Veränderung der Abschattungsfläche bzw. der auf den Lichtempfänger 13 auftreffenden Lichtmenge zweimal pro Zwirnperiode statt, so daß die opto-elektronische Meß- und Auswerteinheit 13, 14 entsprechende Signale bzw. Meßwerte abgibt, die einen gleichmäßigen Zwirn anzeigen.

Wenn durch die Meßstrecke ein ungleichmäßiger Zwirn beispielsweise entsprechend den Figuren 2 und 3 hindurchbewegt wird, resultiert daraus eine sich langsam periodisch ändernde Abschattung des Strahlenbündels einmal pro Zwirnperiode, woraus sich entsprechende Meßwerte ergeben, die das Vorhandensein eines ungleichmäßigen Zwirns anzeigen.

Wie bereits erwähnt hat die Verstellbarkeit bzw. Drehbarkeit der Blende 4 den Zweck, unterschiedliche Drehungs- bzw. Zwirnsteigungswinkel α (entsprechend der Drehungsdichte je Längeneinheit des Zwirns) berücksichtigen zu können, um den Blendenschlitz 5 jeweils in die optimale Stellung in Übereinstimmung mit dem Winkel α parallel zu diesem auszurichten.

Die Blende 4 kann soweit verdreht werden, daß auch S- und Z-gedrehte Zwirne gleichmäßig mit ein und derselben Blende erfasst werden können.

Es ist auch denkbar, in der Blende anstelle eines einzigen Schlitzes einen Kreuzschlitz anzubringen, der die gleiche Aufgabe wie ein einzelner Schlitz erfüllt und ohne große Verstellung auch die Zwirnperiode der Gegenlaufrichtung aufnehmen kann.

Figur 5 zeigt ein Handmeßgerät mit einem Gehäuse 15, in dem in Übereinstimmung mit Figur 4 eine Lichtquelle 10, ein Spiegel bzw. Hohlspiegel 11 und eine Linse 12 als Lichtsender und ein Lichtempfänger 13 in Form einer Fotodiode oder eines Fototransistors untergebracht sind. An den Lichtempfänger 13 ist entweder innerhalb des Handmeßgerätes 15 oder ausserhalb des Handmeßgerätes über ein Kabel 17 ein nicht dargestellter Auswerter angeschlossen. Zwischen dem Lichtsender 10, 11, 12 und dem Lichtempfänger 13 befindet sich die verstellbare Blende 4 mit mindestens einem Blendenschlitz 5 bzw. 5'.

Die Blende 4 ist gemäß Figur 6 an einer Blendenbuchse 4' befestigt, die eine Skala 4'' trägt und an der das Betätigungselement 6 befestigt ist. Die Blende 4 mit der Blendenbuchse 4' sind in einem eine seitliche Aussparrung aufweisenden Blendengehäuse 16 drehbar gelagert, das mit einer mit der Skala 4'' zusammenwirkenden Markierung 16' versehen ist. Diese Blendenkonstruktion dient dazu, um den Blendenschlitz 5 bzw. 5' in einfacher und zuverlässiger Weise in die dem Zwirn - steigungs bzw. Drehungswinkel α des zu untersuchenden Zwirns entsprechende Lage zu verstellen.

Die Blende 4 kann, beispielsweise bezogen auf die Darstellung von Figur 4, entweder zwischen dem zu untersuchenden Zwirn Z und dem Lichtempfänger 13 oder zwischen dem Lichtsender 10, 11, 12 und dem zu untersuchenden Zwirn Z liegen.

Bei fest im Fadenlauf einer zwirnerzeugenden Maschine angebrachten Meßeinheiten kann die Verstellung des Schlitzwinkels auch durch elektromechanische Mittel erreicht werden, in dem Sinne, daß der Schlitzwinkel selbst dem Zwirnsteigungswinkel angepaßt wird, da in diesem Falle das stärkste Signal erhalten wird.

## Patentansprüche

1. Verfahren zum Erfassen von Längenungleichmäßigkeiten der einzelnen Garnkomponenten eines Zwirns, bei welchem Verfahren der Zwirn in seiner Längsrichtung mit konstanter Geschwindigkeit zwischen einem Lichtsender und einem Lichtempfänger einer opto-elektronischen Meß- und Auswerteinrichtung hindurchgeführt wird, die auf Änderungen der Zwirnstruktur in Längsrichtung reagiert und ein Ausgangssignal abgibt, dessen zeitlicher Verlauf eine Funktion der abgetasteten Strukturänderungen darstellt, so daß dieses Ausgangssignal analysiert und auf periodische Komponenten untersucht und daraus die Zwirnperiode und/oder weitere, die Struktur des Zwirns charakterisierende Parameter, bestimmt werden können, dadurch gekennzeichnet, daß man vor oder in das von dem Lichtsender (10, 11, 12) ausgesandte Strahlenbündel eine Schlitzblende (4) einführt und den Schlitz (5 bzw. 5') dieser Blende (4) relativ zur Bewegungsrichtung des Zwirns (Z) schrägstellt.

2. Verfahren nach Anspruch 1, dadurch gekennzeichnet, daß man eine Blende mit zwei sich vorzugsweise rechtwinklig kreuzenden Schlitzen (5,5') verwendet.

3. Verfahren nach Anspruch 1 oder 2, dadurch gekennzeichnet, daß man den Schlitz (5 bzw.5') vorzugsweise übereinstimmend mit dem Zwirnsteigungswinkel α des zu untersuchenden Zwirns parallel zu diesem Winkel α schrägstellt.

4. Verfahren nach Anspruch 1 oder 2, dadurch gekennzeichnet, daß man eine Blende (4) verwendet, bei der jeder Schlitz (5 bzw.5') eine Höhe zwischen dem 1- bis 3-fachen des Zwirndurchmessers des zu untersuchenden Zwirns hat.

5. Verfahren nach Anspruch 1 oder 2, dadurch gekennzeichnet, daß man eine Blende (4) verwendet, bei der jeder Schlitz (5 bzw.5'), bezogen auf die Drehungsperiode des zu untersuchenden Zwirns, eine Breite entsprechend dem 0,05- bis 0,15-fachen der Zwirndrehungsperiode hat.

6. Vorrichtung zum Erfassen von Längenungleichmäßigkeiten der einzelnen Garnkomponenten eines Zwirns, der in seiner Längsrichtung mit konstanter Geschwindigkeit zwischen einem Lichtsender und einem Lichtempfänger einer opto-elektronischen Meß- und Auswerteinrichtung hindurchgeführt wird, die auf Änderungen der Zwirnstruktur in Längsrichtung reagiert und ein Ausgangssignal abgibt, dessen zeitlicher Verlauf eine Funktion der abgetasteten Strukturänderungen darstellt, so daß dieses Ausgangssignal analysiert und auf periodische Komponenten untersucht und daraus die Zwirnperiode und/oder weitere, die Struktur des Zwirns charakterisierende Parameter bestimmt werden können, gekennzeichnet durch eine in den von dem Lichtsender ausgesandten Strahlenbündel liegende Schlitzblende (4), deren Schlitz (5 bzw. 5') relativ zur Bewegungsrichtung des Zwirns schräggestellt ist.

7. Vorrichtung nach Anspruch 6, dadurch gekennzeichnet, daß der Schlitz (5 bzw. 5') einen rechteckigen Querschnitt hat.

8. Vorrichtung nach Anspruch 6, dadurch gekennzeichnet, daß die Blende (4) zwei sich vorzugsweise rechtwinklig kreuzende Schlitze (5,5') hat.

9. Vorrichtung nach Anspruch 8, dadurch gekennzeichnet, daß die Schlitze (5, 5') sich rechtwinklig kreuzen.

10. Vorrichtung nach einem der Anspruch 6-9, dadurch gekennzeichnet, daß die Blende (4) um eine die Längsachse des zwischen dem Lichtsender (10,11,12) und dem Lichtempfänger (13) hindurchlaufenden Zwirns rechtwinklig schneidende Achse verstellbar ist.

11. Vorrichtung nach einem der Ansprüche 6 - 10, dadurch gekennzeichnet, daß sie in einem als Handmeßgerät ausgebildeten Gehäuse (15) untergebracht ist, das einen die Meßstrecke für den zu untersuchenden Zwirns bildenden Schlitz (17) aufweist, der zwischen dem Lichtsender (10,11,12) und dem Lichtempfänger (13) liegt.

12. Vorrichtung nach einem der Ansprüche 8 - 11, dadurch gekennzeichnet, daß die Blende (4) zwischen dem zu untersuchenden Zwirnfaden und dem Lichtempfänger (13) liegt.

13. Vorrichtung nach einem der Ansprüche 8 - 11, dadurch gekennzeichnet, daß die Blende (4) zwischen dem zu untersuchenden Zwirn und dem Lichtsender (10,11,12) liegt.

14. Vorrichtung nach Anspruch 11, dadurch gekennzeichnet, daß die Blende (4) an einer innerhalb eines Blendengehäuses (16) drehbar gelagerten Blendenhülse (4') befestigt ist.

15. Vorrichtung nach Anspruch 14, dadurch gekennzeichnet, daß die Blendenhülse (4') mit einer Skala (4'') versehen ist, während das Blendengehäuse (16) mit einer mit der Skala (4'') zusammenwirkenden Markierung (16') versehen ist.

16. Vorrichtung nach Anspruch 10, dadurch gekennzeichnet, daß die Blende (4) elektromechanisch durch ein von dem Zwirnsteigungswinkel α abgeleitetes Signal verstellbar ist, derart daß die Schrägstellung des Schlitzes (5,5') an den Zwirnsteigungswinkel α angepaßt wird.
